# EUROPEAN PATENT APPLICATION

(11) **EP 4 800 693 A1**
(43) Date of publication of application: **02.09.2026**
(21) Application number: 25160631.5
(22) Date of filing: 27.02.2025
(51) Int. Cl.: G16C 20/80

(54) **DATA STRUCTURE FOR PARTIALLY ELUCIDATED MOLECULAR STRUCTURES**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present disclosure relates to a data structure for partially elucidated structures of chemical compounds and its use.

## Description

### FIELD OF THE DISCLOSURE

The present disclosure relates to a data structure for partially elucidated structures of chemical compounds and its use.

### BACKGROUND

The representation of chemical structures in machine-readable form is crucial for several reasons, significantly impacting research, development, and application across various fields of science and industry.

For example, machine-readable formats enable the efficient storage, retrieval, and sharing of chemical structure information across different platforms and databases. This facilitates collaboration among scientists and allows for the integration of chemical data into larger datasets for comprehensive analysis.

For example, the concise reporting of substances is important for scientific, safety, and regulatory purposes. Depiction of graphical representations of molecular structures provides good readability for human readers, while machine-readable formats allow the use of search algorithms, comparison algorithms, and other automated tools.

Machine-readable chemical structures are essential for computational models that predict the properties, reactivity, and biological activity of molecules. These models play a crucial role in drug design, allowing researchers to simulate interactions between drugs and biological targets to identify promising candidates for further development.

The development of AI and machine learning models for chemical prediction and synthesis planning requires vast amounts of data in machine-readable formats. These models can uncover patterns and relationships that are not immediately apparent, leading to novel insights and innovations.

Chemical structures can be represented in machine-readable form through various formats, each designed to capture different aspects of molecular structure. Examples are SMILES (Simplified Molecular Input Line Entry System), InChI (International Chemical Identifier), and MOL files.

Partially elucidated structures refer to molecules whose overall structure is known to some extent, but not completely. This can occur when certain aspects of a molecule, such as the presence of specific functional groups, and/or the exact arrangement of atoms, e.g. in a complex ring system, remain undetermined due to limitations in analytical techniques and/or sample complexity.

Partially elucidated structures pose a challenge for representation in machine-readable formats due to the uncertainty and/or ambiguity in certain parts of the molecule.

### SUMMARY

The present disclosure provides a data structure for partially elucidated structures.

The data structure comprises:
- a graph defining atoms and bonds between the atoms of a chemical compound, and
- one or more modifiers selected from:
   - substitution modifier
   - addition modifier
   - removal modifier
   - rearrangement modifier
wherein each modifier defines the atoms and/or bonds of the graph affected by the modifier and how they are affected.

In another aspect, the present disclosure relates to the use of the data structure for representing and/or storing and/or processing partially elucidated structures and/or conducting searches and/or calculations based on partially elucidated structures.

In other aspects, the present disclosure provides computer-implemented methods of
generating a data structure based on user input,
enumerating structural variants of a chemical compound,
elucidating a chemical structure of a chemical compound,
updating a data structure of a chemical compound,
and/or searching a query compound.

In another aspect, the present disclosure provides a computer system comprising:
a processing unit; and
a memory storing a computer program configured, when executed by the processing unit, to cause the computer system to perform one or more of the computer-implemented methods of the present disclosure.

In another aspect, the present disclosure provides a non-transitory computer readable storage medium having stored thereon a computer program that, when executed by a processing unit of a computer system, cause the computer system to perform one or more of the computer-implemented methods of the present disclosure.

### BRIEF DESCRIPTION OF THE DRAWINGS

Fig. 1 (a) shows an example of a molecular structure of the chemical compound 2,3,4,5-tetrahydro-4-pyridinol.
Fig. 1 (b) shows the chemical structure of 2,3,4,5-tetrahydro-4-pyridinol without any hydrogen atoms.
Fig. 2 (a) is a graphical representation of a substitution modifier acting on a molecular graph.
Fig. 2 (b) shows one structural variant obtained from the action of the substitution modifier on the molecular graph depicted in Fig. 2 (a).
Fig. 2 (c) shows another structural variant obtained from the action of the substitution modifier on the molecular graph depicted in Fig. 2 (a).
Fig. 3 is another graphical representation of a substitution modifier acting on a molecular graph.
Fig. 4 is a graphical representation of an addition modifier acting on a molecular graph.
Fig. 5(a) is a graphical representation of a removal modifier acting on a molecular graph.
Fig. 5 (b) shows one structural variant obtained from the action of the removal modifier on the molecular graph depicted in Fig. 5 (a).
Fig. 5 (c) shows another structural variant obtained from the action of removal modifier on the molecular graph depicted in Fig. 5 (a).
Fig. 6 (a) and 6 (b) graphically represent a rearrangement modifier. Fig. 6 (a) shows one structural variant obtained from the rearrangement modifier. Fig. 6 (b) shows another structural variant obtained from the rearrangement modifier.
Fig. 7 shows an example of a 1^{st}-order modifier and a 2^{nd}-order modifier.
Fig. 8 (a) shows an example of a chemical compound that can be modified by four modifiers to create structural variants.
Fig. 8 (b) shows one example of a structural variant obtained from applying the modifiers depicted in Fig. 8 (a).
Fig. 9 illustrates a computer system according to some example implementations of the present disclosure in more detail.

### DETAILED DESCRIPTION

Various example embodiments will be more particularly elucidated below without distinguishing between the aspects of the disclosure (data structure, method, computer system, computer-readable storage medium). On the contrary, the following elucidations are intended to apply analogously to all the aspects of the disclosure, irrespective of in which context (data structure, method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless, for example one step builds upon another step, this requiring that the building step be executed subsequently (this being, however, clear in the individual case). The stated orders may thus be exemplary embodiments of the present disclosure.

As used herein, the articles "a" and "an" are intended to include one or more items and may be used interchangeably with "one or more" and "at least one". As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The terms used in this disclosure have the meaning that these terms have in the prior art, in particular in the prior art cited in this disclosure, unless otherwise indicated.

The present disclosure provides a data structure.

A "data structure" is a specialized format for organizing, processing, storing, and/or retrieving data.

The data structure of the present disclosure is machine-readable. The term "machine-readable" refers to a format that can be processed by a computer system without needing human intervention to interpret it. Machine-readable data is structured in such a way that software applications can directly parse, understand, and use it, enabling automated processing, analysis, and/or decision-making.

The data structure of the present disclosure makes partially elucidated structures available for processing by means of a computer system.

The term "partially elucidated structure" refers to molecules whose overall structure is known to some extent, but not completely. The data structure of the present disclosure enables the processing of structures that exhibit a certain degree of uncertainty.

The data structure of the present disclosure comprises a graph.

A graph is a structure comprising a set of objects where some pairs of the objects are in some sense "related". The objects are represented by abstractions called vertices (also called nodes or points) and each of the related pairs of vertices is called an edge (also called link or line).

The graph of the present disclosure defines a molecular structure or sub-structure of a chemical compound: the vertices represent atoms within the molecule; the edges represent chemical bonds between pairs of atoms. This type of graph is also commonly referred to as molecular graph.

Each vertex may be labelled with the element symbol of the corresponding atom (e.g., C for carbon, O for oxygen, N for nitrogen). Edge labels may indicate single, double, triple, or aromatic bonds, depending on the nature of the bond between the atoms.

While many molecular graphs treat edges as unweighted, representing merely the existence of a bond, it is also possible to assign weights to edges. These weights can represent bond order, bond length, and/or other relevant chemical properties.

Most molecular graphs are undirected because the chemical bonds they represent do not have a directionality in the same sense as arrows in a directed graph. However, in certain contexts, directed edges may be used to indicate the direction of electron flow or dipole moments, for example.

Besides the basic structure of atoms and bonds, molecular graphs can be enhanced with additional features such as stereochemistry, to represent three-dimensional molecular structures accurately. Labels may be added to indicate specific isotopes, charge states, and/or other atomic properties.

There are several ways to implement a chemical structure as a molecular graph. It should be noted that the way in which the molecular structure of the chemical compound is implemented as a molecular graph is unimportant. In other words: the data structure of the present disclosure is not limited to specific implementations.

A molecular graph may be represented by one or more matrices, such as an adjacence matrix or by listing for each atom all other atoms it is connected to.

A molecular graph may be represented as a sequence of symbols. An example of such a sequence of symbols is the simplified molecular-input line-entry system (SMILES) representation, which is often stored as an ASCII string.

A molecular graph may be represented as a hierarchy of layers, each layer representing different information of the molecular graph. An example is the IUPAC International Chemical Identifier (InChI), which is often stored as an ASCII string.

A molecular graph may be represented as a MOL file. A MOL file (sometime referred to as MDL MOL file) is a file format for holding information about the atoms, bonds, connectivity and coordinates of a molecule. A MOL file consists of some header information, a connection table containing atom information, bond connections and types, followed by sections for more complex information.

Many software systems/applications in cheminformatics can read and interpret the format; it is also supported by some calculation programs such as Mathematica.

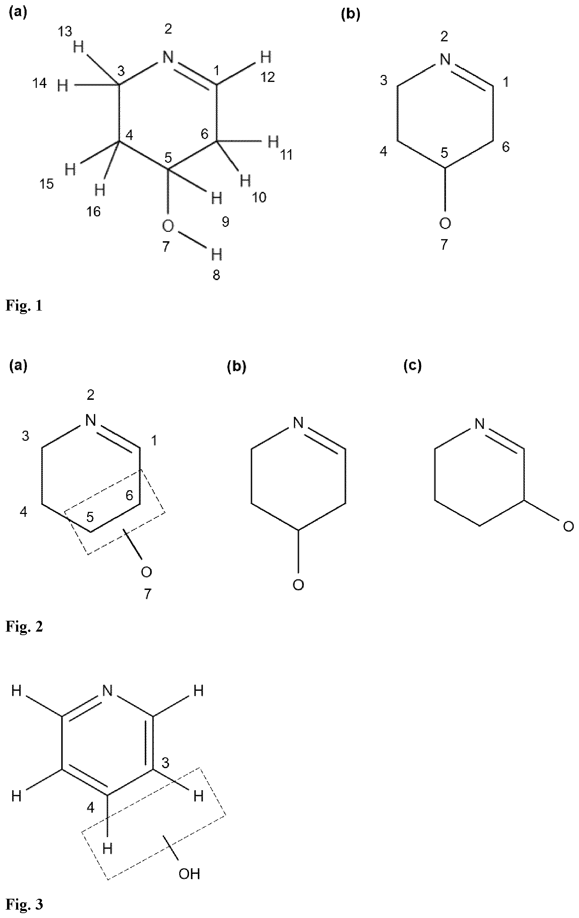

Fig. 1 (a) shows an example of a molecular structure of the chemical compound 2,3,4,5-tetrahydro-4-pyridinol. The InChI code is InChI=1S/C5H9NO/c7-5-1-3-6-4-2-5/h3,5,7H,1-2,4H2. The canonical SMILES code is C1CN=CCC1O.

Table 1 shows an example of an adjacency matrix of 2,3,4,5-tetrahydro-4-pyridinol. For a better overview, the atoms in Fig. 1 (a) have been numbered; these numbers can also be found in the adjacency matrix (in the first row as well as in the first column). The adjacency matrix indicates which atoms are connected to each other. In this example, the number "1" stands for a single bond and the number "2" for a double bond.

**Table 1: Adjacency Matrix of the chemical compound depicted in Fig. 1 (a).**

| | | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|---|
| | | **C** | **N** | **C** | **C** | **C** | **C** | **O** | **H** | **H** | **H** | **H** | **H** | **H** | **H** | **H** | **H** |
| 1 | **C** | | 2 | | | | 1 | | | | | | 1 | | | | |
| 2 | **N** | 2 | | 1 | | | | | | | | | | | | | |
| 3 | **C** | | 1 | | 1 | | | | | | | | | 1 | 1 | | |
| 4 | **C** | | | 1 | | 1 | | | | | | | | | | 1 | 1 |
| 5 | **C** | | | | 1 | | 1 | 1 | | 1 | | | | | | | |
| 6 | **C** | 1 | | | | 1 | | | | | 1 | 1 | | | | | |
| 7 | **O** | | | | | 1 | | | 1 | | | | | | | | |
| 8 | **H** | | | | | | | 1 | | | | | | | | | |
| 9 | **H** | | | | | 1 | | | | | | | | | | | |
| 10 | **H** | | | | | | 1 | | | | | | | | | | |
| 11 | **H** | | | | | | 1 | | | | | | | | | | |
| 12 | **H** | 1 | | | | | | | | | | | | | | | |
| 13 | **H** | | | 1 | | | | | | | | | | | | | |
| 14 | **H** | | | 1 | | | | | | | | | | | | | |
| 15 | **H** | | | | 1 | | | | | | | | | | | | |
| 16 | **H** | | | | 1 | | | | | | | | | | | | |

In organic chemistry, hydrogen atoms are often omitted from chemical structures but defined implicitly by valency rules. Sometimes hydrogen atoms attached to aromatic carbon atoms are omitted, sometimes hydrogen atoms attached to all carbon atoms are omitted, sometimes all hydrogen atoms are omitted. The omission of hydrogen atoms serves to provide a better overview. Fig. 1 (b) shows the chemical structure of 2,3,4,5-tetrahydro-4-pyridinol without any hydrogen atoms.

Likewise, hydrogen atoms are often not included in data structures that represent chemical compounds. For example, the SMILES code of the chemical compound shown in Fig.1 (a) does not include hydrogen atoms. Likewise, the hydrogen atoms in the adjacency matrix shown in Table 1 can be omitted. Table 2 shows the adjacency matrix of the chemical compound depicted in Fig. 1 (b).

**Table 2: Adjacency Matrix of the chemical compound depicted in Fig. 1 (b).**

| | | 1 | 2 | 3 | 4 | 5 | 6 | 7 |
|---|---|---|---|---|---|---|---|---|
| | | **C** | **N** | **C** | **C** | **C** | **C** | **O** |
| 1 | **C** | | 2 | | | | 1 | |
| 2 | **N** | 2 | | 1 | | | | |
| 3 | **C** | | 1 | | 1 | | | |
| 4 | **C** | | | 1 | | 1 | | |
| 5 | **C** | | | | 1 | | 1 | 1 |
| 6 | **C** | 1 | | | | 1 | | |
| 7 | **O** | | | | | 1 | | |

A software that processes a data structure not containing hydrogen atoms, e.g. a software used to calculate chemical and/or physical properties of a chemical compound based on the chemical structure of the chemical compound, usually is configured to supplement free valences of atoms with hydrogen atoms. The data structure of the present disclosure may include hydrogen atoms; however, it is also possible that hydrogen atoms are not directly represented in the data structure.

The adjacency matrices shown in Tables 1 and 2 contain vertices (atoms) of a (molecular) graph and edges (chemical bonds) between the vertices (atoms).

It is also possible to represent vertices (atoms) and edges (chemical bonds) separately. An example of such a separate representation is a MOL file representation.

A portion of a MOL file representation of the chemical compound shown in Fig. 1 (b), is:

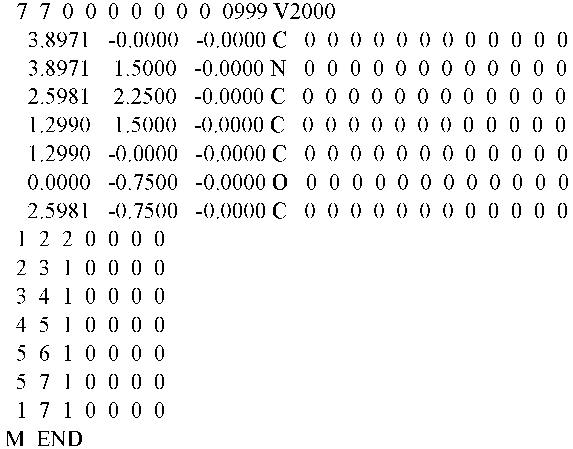

The MOL file representation comprises two blocks: an atom block and a bonding block.

The atom block lists all the atoms in the molecule (hydrogen atoms are omitted). Each line in the atom block represents a single atom, with fields for the atom's x-, y- and z-coordinates, the element symbol, and possibly additional data such as charge and/or stereochemistry.

The bond block follows the atom block. The bond block lists all the bonds between the atoms. Each line represents a bond, with fields indicating the indices of the two atoms involved in the bond, the bond type (e.g., single, double), and possibly stereochemistry information.

For example, the first row of the bond block (12 2 0 0 0 0) indicates that the atom defined in the 1^{st} row of the atom block (C atom) is connected to the atom defined in the 2^{nd} row of the atom block (N atom) by a double bond. For example, the second row of the bond block (2 3 1 0 0 0 0) indicates that the atom defined in the 2^{nd} row of the atom block (N atom) is connected to the atom defined in the 3^{rd} row of the atom block (C atom) by a single bond.

For the sake of simplicity, the data structure of the present disclosure is explained below using a simplified MOL file representation, without the intention of limiting the disclosure to such a representation. An expert in cheminformatics knows of how to apply the following explanations to other representations.

In this simplified MOL file representation, only the atoms are defined in an atom block, and the bonds between the atoms are defined in a bond block. To save space, the atoms block is displayed as a row. The following is a simplified MOL file representation for the chemical compound shown in Fig. 1(b):

| C | N | C | C | C | O | C |
|---|---|---|---|---|---|---|
| 1 | 2 | 2 | | | | |
| 2 | 3 | 1 | | | | |
| 3 | 4 | 1 | | | | |
| 4 | 5 | 1 | | | | |
| 5 | 6 | 1 | | | | |
| 5 | 7 | 1 | | | | |
| 1 | 7 | 1 | | | | |

It is possible that the molecular graph of the data structure of the present disclosure contains information for each atom and/or each bond indicating whether the atom and/or bond is considered certain or whether the atom and/or bond is associated with an uncertainty. It is also possible that only atoms and/or bonds that are considered certain are labelled, or that only atoms and/or bonds that are considered uncertain are labelled.

"Certain" means that it is assumed that an atom and/or a bond in the molecule actually occurs as defined by the molecular graph. "Uncertain" means that an atom and/or a bond may differ from the molecular graph.

Fig. 2(a) shows an example of a chemical compound in which the oxygen atom 7 can be attached to the carbon atom 5 or to the carbon atom 6. The atoms with the indices 1, 2, 3 and 4 as well as the bonds between them are considered "certain". The bond between the oxygen atom 7 and the carbon atom 5 is "uncertain". Likewise, the bond between oxygen atom 7 and carbon atom 6 is "uncertain". "Certainty" or "uncertainty" can be expressed, for example, using categorical variables. For example, certainty can take the value 1 and uncertainty can take the value 0.

It is also possible to define how certain atoms and/or bonds are. This can be done, for example, by means of a certainty score. The certainty score may, for example, take the value 1 if an atom and/or a bond is considered certain, and may take a value between 0 and 1 if the atom and/or bond is subject to uncertainty, where the value can be smaller the greater the uncertainty.

Whether an atom and/or a bond is considered "certain" or "uncertain" or how "certain" the atom and/or the bond is, can be determined by a user. It is also possible that this information is automatically derived from an analysis result. If the molecular structure of the chemical compound under consideration is a molecular structure proposed and/or generated and/or predicted by an artificial intelligence system, this information can also be provided by the artificial intelligence system.

The data structure of the present disclosure further comprises one or more modifiers. The number of modifiers may be one or two, or three, or four, or five, or six, or seven, or eight, or nine, or ten, or more than ten. Usually (but not necessarily), the number of modifiers is smaller than the number of atoms in the molecular graph.

A modifier refers to one or more atoms and/or one or more bonds in the molecular graph. A modifier defines the atom(s) and/or bond(s) of the graph affected by the modifier. The modifier may list the atom(s) and/or bond(s) affected by the modifier.

If a modifier refers to an atom and/or a bond, this means that the atom/bond is subject to uncertainty.

The modifier indicates that the atom/bond can be different than defined in the molecular graph.

The modifier indicates how the atom/bond may be modified.

A modifier modifies the molecular graph of the data structure. The modifier defines the modification that is made to the molecular graph. The modifier can be used to generate structural variants based on the molecular graph. These structural variants are possible structural variants of the chemical compound represented by the data structure.

A plurality of structural variants can be generated from the molecular graph by combinational application of modifiers. The structural variants do not have to be listed individually but can be derived from the combinational application of the modifier on the molecular graph.

Various modifiers are defined in the data structure of the present disclosure.

One example of a modifier is a substitution modifier. In case of a substitution modifier, an atom or a group of atoms is replaced by another atom or another group of atoms. A "group of atoms" is a molecular fragment that has a binding site with which it can be connected to an atom of the molecular graph.

For example, it is possible that a hydrogen atom of the molecular graph may be replaced by a chlorine atom or a hydroxyl group (OH group).

It is possible that the substitution modifier indicates the atom that may be replaced by another atom and/or an atomic group.

However, since hydrogen atoms (in particular hydrogen atoms attached to carbon atoms) are often omitted in molecular graphs and associated data structures, the substitution modifier may also specify the atom to which a substituted and/or to be substituted atom or group of atoms is attached.

Usually, several atoms in the molecular graph are affected by a substitution modifier: the substituent can be attached at different points in the molecular graph; each of these points is a point affected by the substitution modifier.

Fig. 2 shows an example. Fig. 2(a) shows an example of a chemical compound in which the oxygen atom 7 can be attached to the carbon atom 5 or to the carbon atom 6. When oxygen atom 7 is bonded to carbon atom 5, the structure shown in Fig. 2(b) is obtained. When oxygen atom 7 is bonded to carbon atom 6, the structure shown in Fig. 2(c) is obtained.

Note that the atoms in Fig. 2 (a) have been arbitrarily indexed. Note that the data structure of the present disclosure is not tied to any convention (e.g. IUPAC rules) for numbering atoms.

The molecular graph representing the chemical structures depicted in Fig. 2 comprises the molecular graph of 1-piperideine:

| C | N | C | C | C | C |
|---|---|---|---|---|---|
| 1 | 2 | 2 | | | |
| 2 | 3 | 1 | | | |
| 3 | 4 | 1 | | | |
| 4 | 5 | 1 | | | |
| 5 | 6 | 1 | | | |
| 1 | 6 | 1 | | | |

The molecular graph of 1-piperideine is modified by a substitution modifier. The substitution modifier affects carbon atom 5 and carbon atom 6. An example of a substitution modifier is:
"substitution modifier"
5, 6
-H, +O

In the first row, the substitution modifier specifies the carbon atoms 5 and 6 at which a substitution occurs. In the second row, the substitution modifier specifies which atom is replaced (H) and by which atom the replaced atom is replaced (O). For the sake of illustration, it was specified here that a hydrogen atom is removed (-H) and an oxygen atom is added (+O). However, it is of course possible to omit both + and -.

Likewise, it is possible to omit the letter H if it is obvious that a substitution of a hydrogen atom is taking place.

The example of a substitution modifier described here was based on a molecular graph that does not include hydrogen atoms. The atoms affected by the substitution modifier were specified as the carbon atoms at which the substitution occurs. In the case that a molecular graph explicitly includes hydrogen atoms, the substitution modifier may also directly specify which atoms or groups of atoms are substituted.

This is explained in more detail in relation to Fig. 3. Fig. 3 shows a pyridine molecule which is substituted in either position 3 or 4 with a hydroxyl (OH) group. The data structure representing the compound depicted in Fig. 3 may comprise the following molecular graph of pyridine:

| N | C | C | C | C | C | H | H | H | H | H |
|---|---|---|---|---|---|---|---|---|---|---|
| 1 | 5 | 2 | | | | | | | | |
| 1 | 6 | 1 | | | | | | | | |
| 2 | 3 | 2 | | | | | | | | |
| 2 | 4 | 1 | | | | | | | | |
| 2 | 7 | 1 | | | | | | | | |
| 3 | 5 | 1 | | | | | | | | |
| 3 | 8 | 1 | | | | | | | | |
| 4 | 6 | 2 | | | | | | | | |
| 4 | 9 | 1 | | | | | | | | |
| 5 | 10 | 1 | | | | | | | | |
| 6 | 11 | 1 | | | | | | | | |

An example of a substitution modifier is:
"substitution modifier"
8, 9
*OH

According to the molecular graph of pyridine, hydrogen atoms 8 and 9 are bonded to carbon atoms 3 and 4. In the first row, the substitution modifier specifies the hydrogen atoms that may be substituted (8 and 9). In the second row, the substitution modifier specifies the atom or group of atoms by which the hydrogen atoms may be substituted; in the example at hand, this is an OH group, with * indicating that the bond to the carbon atom is via the O atom.

It should be noted that a substitution modifier may also be realized by a combination of a removal modifier and an addition modifier (as defined below). However, the substitution modifier usually represents a higher certainty and usually leads to a higher identification confidence level. Furthermore, a substitution modifier is usually easier to implement in cheminformatics toolkits. It can therefore be advantageous to use a substitution modifier rather than a combination of a removal modifier and an addition modifier.

Another example of a modifier is an addition modifier. In the case of an addition modifier, an atom or a group of atoms is a component of the molecule, but this atom or group of atoms is not a component of the molecular graph and is therefore included by an addition modifier.

In other words, it is certain that the molecule includes an atom or group of atoms; however, it is unclear where this atom or group of atoms occurs in the molecule, so it is not included in the molecular graph. Instead, it is attached to the molecular graph using an addition modifier. The addition modifier indicates the positions at which the atom or group may be attached to the molecular graph. In contrast to the substitution modifier, an addition modifier does not replace an atom with another atom or group of atoms, but an atom or a group of atoms is added to the molecular graph. In contrast to the substitution modifier, it can also happen that atoms from the addition modifier are added to multiple atoms and that the added atoms are not bonded directly to each other.

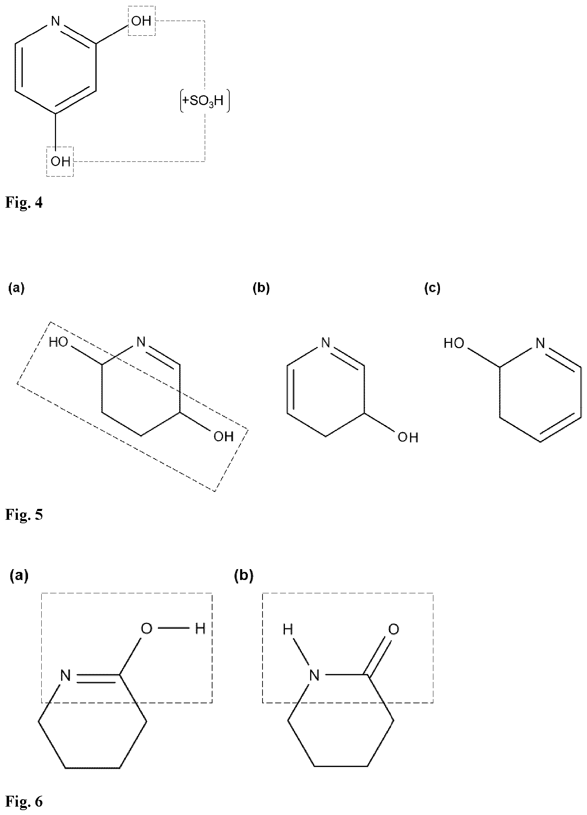

Fig. 4 shows an example. Fig. 4 shows 2,4-dihydroxypyridine to which an SO₃H group is added. It is unclear to which hydroxyl group the SO₃H group is added. An example of a data structure for the chemical compound depicted in Fig. 4 is:

| N | C | C | C | C | C | O | O |
|---|---|---|---|---|---|---|---|
| 2 | 3 | 1 | | | | | |
| 3 | 4 | 2 | | | | | |
| 4 | 5 | 1 | | | | | |
| 5 | 6 | 2 | | | | | |
| 6 | 7 | 1 | | | | | |
| 4 | 8 | 1 | | | | | |
| 1 | 2 | 2 | | | | | |
| 6 | 1 | 1 | | | | | |

"addition modifier"
7, 8
+SO₃H

The data structure comprises a molecular graph of 2,4-dihydroxypyridine. According to the molecular graph, the oxygen atoms carry the indices 7 and 8. In the first row, the addition modifier specifies that the oxygen atoms 7 and 8 are affected by the addition modifier. In the second row, the addition modifier specifies that an SO₃H is added.

Another example of an addition modifier is the addition of a water molecule (+H₂O) where one H atom might be added to a different atom than the OH.

Another example is "+O", which may mean insertion of an O atom into a C-H bond, or addition to an N atom (or an S atom) to form an N-oxide (or sulfoxide).

It may also happen that bonds defined in the molecular graph are broken upon addition of atoms, which can happen with +H₂O for example. Therefore, the addition modifier represents a higher degree of uncertainty than the substitution modifier.

Another example of a modifier is a removal modifier. Removal is the opposite of addition. In the case of a removal modifier, an atom or a group of atoms is removed from the molecular graph. The removal modifier indicates at which points of the molecular graph the atom or group of atoms may be removed, and which atom or group of atoms may be removed.

Fig. 5 shows an example. The chemical compound shown in Fig. 5(a) has lost a water molecule (-H₂O); it is unclear where the water molecule came from. There are two possibilities for the loss of a water molecule: the resulting structures are shown in Fig. 5(b) and Fig. 5(c).

An example of a data structure for the chemical compound shown in Fig. 5 is:

| N | C | C | C | C | C | O | O |
|---|---|---|---|---|---|---|---|
| 2 | 3 | 1 | | | | | |
| 3 | 4 | 1 | | | | | |
| 4 | 5 | 1 | | | | | |
| 5 | 6 | 1 | | | | | |
| 1 | 2 | 1 | | | | | |
| 1 | 6 | 2 | | | | | |
| 5 | 7 | 1 | | | | | |
| 2 | 8 | 1 | | | | | |

"removal modifier"
2, 3, 4, 5, 7, 8
-OH, -H

In the first row, the removal modifier specifies the atoms which may be affected by the removal modifier; in the second row, the removal modifier specifies that an OH-group and a hydrogen atom is removed from the molecule represented by the molecular graph (resulting in the removal of a water molecule).

Another example of a modifier is rearrangement modifier. The rearrangement modifier changes the position of atoms or groups of atoms in the molecular graph. The rearrangement modifier can cause one or more atoms or groups of atoms to swap positions within the molecular graph. The rearrangement modifier can indicate which atoms or groups of atoms may swap positions. Such an exchange of atoms or groups of atoms may also cause bond changes within the molecular graph.

Fig. 6 shows an example. The chemical compound shown in Fig. 6 can have the structure shown in Fig. 6(a) or that shown in Fig. 6(b). The dashed frame marks the atoms and bonds that may be rearranged. An example of a data structure of the chemical compound depicted in Fig. 6 is:

| N | C | C | C | C | C | O |
|---|---|---|---|---|---|---|
| 2 | 3 | 1 | | | | |
| 3 | 4 | 1 | | | | |
| 4 | 5 | 1 | | | | |
| 5 | 6 | 1 | | | | |
| 1 | 2 | 1 | | | | |
| 1 | 6 | 2 | | | | |
| 6 | 7 | 1 | | | | |

"rearrangement modifier"
1, 6, 7

The rearrangement modifier marks the atoms that can be affected by the modifier.

It is possible that there are further modifiers.

Another modifier may be a stereochemistry modifier. The stereochemistry modifier may indicate a stereocenter and indicate how it can be modified.

A stereocenter may be indicated in the data structure, for example, by the atomic designation and/or a label. For example, stereocenters may be indicated by the use of "@" symbols in the atom block or any other symbol. For example, C@ may indicate that the carbon atom is a stereocenter; C@@ may indicate the opposite configuration (the enantiomer).

Bonds connected to a stereocenter may also specified. The connectivity may indicate the arrangement of substituents around the stereocenter. This may be done by listing the atoms and their connections in the bond block.

The stereochemistry modifier may indicate an atom representing a stereocenter and/or bonds attached to the atom and may indicate one or more operations through which the stereocenter is transformed into its opposite.

Another modifier may be an isotope modifier. The isotope modifier may indicate an atom and indicate which isotopes the atom can represent.

Another modifier may be an annotation modifier. Such an annotation modifier may allow a user, for example, to place free text in a graphical representation of a chemical structure. The annotation modifier may specify at which points (e.g. coordinates) the text is to be placed and which text is to be placed.

Instead of a freely selectable text, a selection may also be available from which a user can choose. Instead of or in addition to text, other graphical elements can also be used as annotations, such as arrows, mathematical symbols and/or the like.

In an embodiment of the present disclosure, modifiers themselves can be the subject of a modification. In other words, modifiers can not only define modifications of the molecular graph, but also modifications of modifiers.

A modifier indicating a modification of the molecular graph is also referred to as a first-order modifier in this disclosure. A modifier indicating a modification of a first-order modifier is also referred to as a second-order modifier in this disclosure. A modifier indicating a modification of a second-order modifier is also referred to as a third-order modifier in this disclosure. And so on.

The number of orders is not limited but is usually less than five.

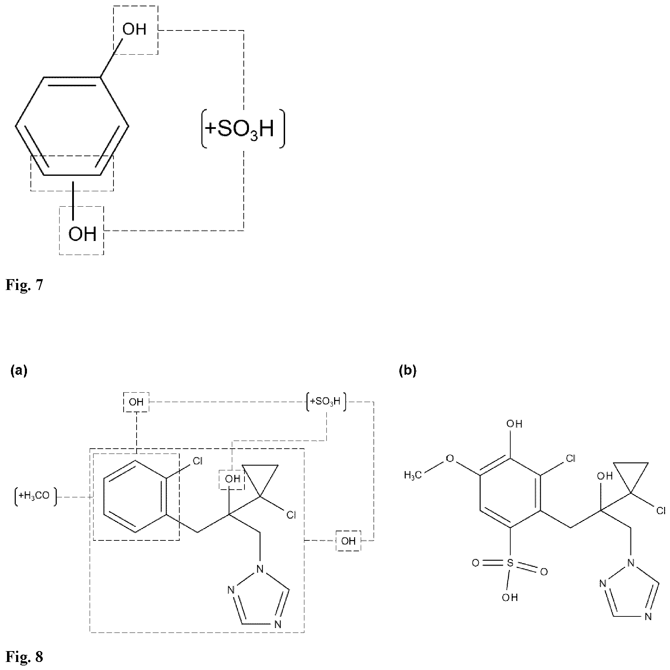

Fig. 7 shows an example with a first-order modifier and a second-order modifier. Fig. 7 shows phenol, which carries another OH group in the meta or para position. In addition, the molecule carries an SO₃H group, which can be added to one of the two OH groups.

The chemical compound shown in Fig. 7 can be represented by a molecular graph of phenol and two modifiers. The first modifier is a substitution modifier and acts on the carbon atoms in the meta and para positions to the OH group. The second modifier is an addition modifier; it acts directly on the OH group and on the OH group added by the first modifier. In the example shown, the substitution modifier is a first-order modifier, and the addition modifier is a second-order modifier.

An example of a data structure of the chemical compound depicted in Fig. 7 is:

| C | C | C | C | C | C | O |
|---|---|---|---|---|---|---|
| 1 | 2 | 2 | | | | |
| 2 | 3 | 1 | | | | |
| 3 | 4 | 2 | | | | |
| 4 | 5 | 1 | | | | |
| 5 | 6 | 2 | | | | |
| 6 | 1 | 1 | | | | |
| 1 | 7 | 1 | | | | |

"substitution modifier"
3, 4
-H, +OH
"addition modifier"
7, OH
+SO₃H

Fig. 8(a) shows an example of a chemical compound that can be modified by four modifiers to create structural variants. Fig. 8(b) shows one example of these structural variants. Annex A provides a detailed data structure for the chemical compound shown in Fig. 8(a). The data structure is based on the V2000 standard for MOL files. This is an example of how the data structure of the present disclosure can be implemented in detail.

The data structure of the present disclosure may comprise further information.

For example, the data structure of the present disclosure may comprise a molecular formula of the chemical compound. A molecular formula indicates the numbers of each type of atom in a molecule of the chemical compound. For example, the molecular formula of the chemical compound depicted in Fig. 1(a) is C₅H₉NO.

This information can be used as a cross-check when a user creates and/or modifies a data structure. For example, if another modifier is added to the data structure, the changes resulting from the modifier should not change the molecular formula. In other words, in a data structure of the present disclosure, the molecular graph together with all modifiers must be consistent with the molecular formula.

The data structure of the present disclosure may comprise a molecular mass of the chemical compound. The molecular mass is the mass of a given molecule. For example, the molecular mass of the compound depicted in Fig. 1(a) is 99.131 amu. This information can be used as a cross-check when a user creates and/or modifies a data structure. For example, if another modifier is added to the data structure, the changes resulting from the modifier should not change the molecular mass. In other words, in a data structure of the present disclosure, the molecular graph together with all modifiers must be consistent with the molecular mass.

The data structure of the present disclosure may comprise an identification confidence level. The term "identification confidence level" refers to a measure or estimation of how certain one can be about the correct identification of the chemical structure represented by the data structure. While the certainty score described above refers to individual atoms and/or bonds, the identification confidence level refers to the complete structure of the chemical compound.

Such an identification confidence level may help to quantify the reliability of the identification process, taking into account the methods used, the quality of the data, and/or the comparison with known standards and/or databases. It may provide a standardized way to communicate the certainty of identification results, guiding further experimental and/or analytical efforts and/or informing the interpretation of results.

In some areas standardized identification confidence levels are used to facilitate communication and/or comparison of results.

For example, in metabolomics, the Metabolomics Standards Initiative (MSI) has proposed a tiered system of identification confidence levels (see, e.g., L.W. Sumner et al.: Proposed minimum reporting standards for chemical analysis, Metabolomics 3, 2007, 211-221; A.C. Schrimpe-Rutledge et al.: Untargeted Metabolomics Strategies - Challenges and Emerging Directions, J. Am. Soc. Mass Spectrom., doi: 10.1007/s13361-016-1469-y).

Schymanski *et al.* proposed a framework for expressing the confidence level of compound identification in environmental sciences, particularly focusing on non-targeted analysis where the identification of unknown chemicals is a common challenge (see, e.g., Schymanski et al.: Identifying Small Molecules via High Resolution Mass Spectrometry: Communicating Confidence, Environ. Sci. Technol., doi: 10.1021/es5002105).

So, the data structure may, for example, comprise an MSI identification confidence level and/or a Schymanski confidence level and/or any other identification confidence level for the chemical compound represented by the data structure.

In an embodiment of the present disclosure, the data structure includes a level of definition. Such a level of definition is an indicator of the variability in the molecular structure, e.g., ranging from Level A (exact structure) to Level E (defined mass), as shown in the table below. These levels of definition represent different information than the identification confidence levels, as they do not refer to any experiment but are defined by the degree of variability in the structure definition itself. An alternative term is the term degree of elucidation.

| **Level** | **Description** | **Modifications allowed** | **Representative structure can be defined?** | **Can be enumerated** |
|---|---|---|---|---|
| Level A: **exact structure** | **Exact structure**, only one possibility | core stereoisomers isotopes | - | - |
| Level B: **Defined functional groups;** | Core structure and **functional groups are** | core stereoisomers substitutions isotopes | | |
| **Positional Isomers** | **defined**, but their position is uncertain. | | ✔ | ✔ |
| Level C: **Scaffold with enumerated additions** | Defined core structure with **addition and/or removal of atoms**. Bond changes only at the periphery of the scaffold. **A list of possible structures can be provided**. | core stereoisomers substitutions additions isotopes | ✔ | ✔ |
| Level D: **Defined Sum Formula** | Only **sum formula** is defined (incl. definition of isotope labels). For labelled studies the atom balance as the parent core structure with **addition and/or removal of atoms** can be provided, which includes other bond changes. | core (optional) stereoisomers substitutions additions removals rearrangements isotopes | ✔ | × |
| Level E: **Defined Mass** | Only the **mass** is provided. | core and modifications can NOT be defined | × | × |

The table shows that the data structure of the present disclosure is particularly suitable for representing chemical compounds of Levels B, C, and D.

The data structure of the present disclosure may comprise information about a review process to which the chemical compound represented by the data structure is subject.

It is possible that the results of an analysis of a chemical compound lead to an initial version of a data structure that includes an initial estimate of the structure of the chemical compound. This first version may comprise the information that the chemical structure represented by the data structure is an initial estimate. This version of the data structure can be shared with one or more other users, e.g. experts, who can check the initial estimate and revise it if necessary. The result of such revision can be a second version of the data structure. This second version can include information that it includes a revised chemical structure. And so on. It is possible to keep earlier versions to enable version control.

Such review information may include information about the stage of a review process. Such review information may include information about an outcome of the review process.

The data structure may contain information on whether and/or how confidential the chemical compound represented by the data structure is. For example, if the chemical compound is a metabolite of a new active ingredient for a drug or a crop protection product, it may be possible to deduce the chemical structure of the active ingredient from the metabolite. To protect the active ingredient, the data structure representing the metabolites can be labelled as confidential. The data structure may include a level of confidentiality indicating how confidential the chemical compound represented by the data structure is.

The data structure of the present disclosure may be created based on user input. Such input is typically made into the computer system of the present disclosure. Typically, such input is made via a graphical user interface.

The graphical user interface may be used to give a user access to a molecule editor. Such molecule editors allow the creation of graphical representations of molecules. The following website provides examples of freely or commercially available molecule editors: https://en.wikipedia.org/wiki/ Molecule_editor.

The user may enter a chemical structure of a chemical compound into the molecule editor using, for example, a mouse and/or keyboard and/or touchpad and/or any other means for entering data into a computer system. This chemical structure may be the basis for the molecular graph; in other words, the computer system may be configured to translate the chemical structure entered by the user into a molecular graph and to write the molecular graph into the data structure.

The user may specify by input which atoms and/or bonds are to be subject to a modification. The user may specify by input how the atoms and/or bonds may be modified. These inputs can be translated by the computer system into modifiers and written into the data structure.

So, another subject of the present disclosure is a computer-implemented method of generating a data structure based on user input. The method comprises:
- receiving or providing a molecular graph;
- receiving an input from a user, the input specifying (i) atoms and/or bonds that are subject to one or more modifications, and (ii), information on the type of modification(s);
- generating a data structure according to the present disclosure based on the molecular graph and the user input.

The computer system may be configured to determine a molecular formula and/or a molecular mass based on the information entered by the user.

The user may enter a measured molecular formula and/or a measured molecular mass, which may have been determined in the course of analyses on the chemical compounds, into the computer system. It is possible for the user to enter other/further measured data into the computer system. It is possible that measurement data is transferred from an analyzer (e.g., mass spectrometer, NMR) to the computer system and/or read out from a data storage device.

The computer system may be configured to compare measured data with data determined by the computer system.

The computer system may be configured to display a warning to the user if measured data and data determined by the computer system differ. Based on such a warning, the user may recognize whether the chemical structure entered by the user is compatible with the measured data.

Measured data may also be written into the data structure.

The user may enter further data into the computer system, such as one or more certainty scores for one or more atoms and/or bonds, an identification confidence level, a level of definition, a confidentiality level, a review level and/or other/further data. Such further data may also be written into the data structure.

The data structure of the present disclosure typically includes a plurality of variations (modifications) of the chemical structure of a chemical compound. For example, if the data structure includes a molecular graph and a substitution modifier that specifies that each of two hydrogen atoms of the molecular graph can be replaced by a different atom or group of atoms (e.g., by an OH group), then the data structure already represents two variants (modifications). If the data structure includes, for example, another modifier that specifies that each of three further hydrogen atoms of the molecular graph can be replaced by another atom or group of atoms (e.g. by an F atom), then the data structure already represents 3*2=6 variants.

The data structure of the present disclosure is able to represent a variety of combinational variations in a simple and memory-efficient way.

Based on the data structure of the present disclosure, all variations of the chemical structure of the chemical compound can be easily and quickly generated. The molecular graph may represent a first structural variant of the chemical compound. All other variants may be generated by simply applying the modifiers one after the other to the atoms and/or bonds of the molecular graph (or other modifiers) that are affected by the respective modifier, taking into account combinations of modifiers.

In other words: based on the data structure of the present disclosure, possible structural variants of the chemical compound covered by the data structure can be generated and listed. Such a list is also referred to as an "enumeration" in this disclosure.

The process of enumerating the structural variants can be done automatically. "Automatically" means without human intervention.

Another subject of the present disclosure is a computer-implemented method of enumerating structural variants of a chemical compound. The method comprises:
- providing a data structure according to the present disclosure, wherein the data structure represents the chemical compound,
- generating structural variants of the chemical compound based on the data structure,
- outputting and/or saving the structural variants and/or transmitting the structural variants to a separate computer system.

Such enumeration of structural variants of a chemical compound may be useful for further elucidating the structure of the chemical compound. For example, all structural variants may be generated, and chemical and/or physical properties may be computed based on the variants created. The computed properties may be compared with measured properties of the chemical compound. In this way, one or more variants may be excluded and the data structure refined.

For example, chemical and/or physical properties can be calculated based on the molecular structure of each variant. There are numerous methods for calculating chemical and/or physical properties based on molecular structure. These can be found in the literature, e.g., under the name QSPR (quantitative structure property relationship). There are also commercially and freely available computer programs for calculating chemical and/or physical properties of chemical compounds based on their molecular structure (e.g..: QSAR-Co: J. Chem. Inf. Model. 2019, 59, 6, 2538-2544; Molgen-QSPR: https://www.researchgate.net/publication/266470632; RDKit: https://www.rdkit.org).

Such chemical and/or physical properties can be, for example, molecular mass, molecular formula, water solubility, partition coefficient (e.g., for octanol/water), melting point (e.g., at standard conditions), pKa/pKb values, pH value in an aqueous solution and/or other/further properties. Such chemical and/or physical properties can also be a computed NMR (nuclear magnetic resonance) spectrum or any other spectrum.

Another subject of the present disclosure is therefore a computer-implemented method of elucidating a chemical structure of a chemical compound. The method comprises:
- providing a data structure according to the present disclosure, wherein the data structure represents the chemical compound,
- generating structural variants of the chemical compound based on the data structure,
- for each structural variant: computing chemical and/or physical properties based on the structural variant,
- comparing computed chemical and/or physical properties with measured chemical and/or physical properties of the chemical compound,
- selecting and/or discarding structural variants based on the result of the comparison.

When comparing a computed property with a measured property, a deviation between the computed property and the measured property is usually determined and quantified.

It is possible that a maximum deviation has been defined for one or more properties. If the computed property deviates from the measured property by more than the maximum deviation, it is unlikely that the chemical compound has the structure defined by the corresponding structural variant. Such a structural variant can be rejected. Each rejected structural variant reduces the number of possible structural variants. It is possible that the deviations of several properties (e.g. two or three or more than three) must be greater than the corresponding defined maximum deviations for a structural variant to be discarded. Maximum deviations may have been set by an expert. It is possible that the user himself/herself sets maximum deviations. It is possible that the user has the possibility to change default values of maximum deviations and/or other/further pre-defined values.

It is possible that one or more structural variants are displayed to the user together with the computed and measured properties (e.g., on a monitor). It is possible that a user is shown deviations between computed and measured properties. It is possible for the user to select and/or discard structural variants.

The results of the calculations and comparisons can help to narrow down the number of possible structural variants and can provide clues as to further investigations that can be carried out to fully elucidate the structure of the chemical compound.

Another subject of the present disclosure is a computer-implemented method for updating a data structure of a chemical compound. The method comprises:
- providing a data structure according to the present disclosure, wherein the data structure represents the chemical compound,
- providing one or more measured chemical and/or physical properties of the chemical compound,
- computing one or more chemical and/or physical properties of one or more structural variants of the chemical compound based on the data structure,
- outputting
   ∘ the one or more computed chemical and/or physical properties and the one or more measured chemical and/or physical properties, and/or
   ∘ one or more deviations between the one or more computed chemical and/or physical properties and the one or more measured chemical and/or physical properties,
- receiving a selection and/or an exclusion by a user,
- updating the data structure based on the user's selection/exclusion,
- outputting and/or saving the updated data structure and/or transmitting the data structure to a separate computer system.

The user's exclusion may comprise an exclusion of one or more structural variants. These structure variants no longer need to be covered by the data structure. The data structure may be changed (updated) so that the excluded structure variants are no longer included. This can be done by deleting and/or changing the modifiers that generate/represent these structural variants. It is also possible that the molecular graph of the data structure is changed.

The user's selection may comprise a selection of one or more structural variants. These structural variants are still possible structural variants of the chemical compound. The data structure may be changed (updated) so it only covers the selected structural variants. This can be done by deleting the modifiers and/or changing the molecular graph in the data structure.

The data structure of the present disclosure further allows a search for a defined chemical structure and/or substructure and/or similar structures and/or substructures.

Since the data structure of the present disclosure already includes a core of a chemical compound in the form of a molecular graph, searches can be carried out based on this molecular graph.

The search options are explained using examples, without the intention of limiting the present disclosure to these examples.

Exact matching is the simplest form of search where the goal is to find an exact match between the molecular graph of the data structure and the molecular graph of a query compound. Exact matching usually involves comparing the query compound's graph to the graph of the data structure by looking at the vertices (atoms) and edges (bonds) to see if the structure is identical. Algorithms like graph isomorphism can be used for this purpose, for example.

Graph isomorphism is a computational task of determining whether two finite graphs are isomorphic. Two graphs (Q) and (C) are isomorphic if there is a bijection (one-to-one correspondence) between the vertex sets of (Q) and (C) that preserves the adjacency relationship. In other words: if it is possible to relabel the vertices of graph (Q) to get graph (C) without changing which vertices are connected, then (Q) and (C) are isomorphic.

One example of a graph isomorphism algorithm is the Weisfeiler-Lehman algorithm. The Weisfeiler-Lehman algorithm is an iterative procedure that refines the classification of vertices based on the principle that two vertices are similar not only if they have the same degree but also if their neighborhoods are structurally similar. Although originally not designed solely for graph isomorphism, it can be used as part of a graph isomorphism test (see, e.g., N. Huang, S. Villar: *A Short Tutorial on The Weisfeiler-Lehman Test And Its Variants*, arXiv:2201.07083v2).

After an exact match between the molecular graph of the query compound and the molecular graph of the data structure has been checked, the same can be done for the structural variants that result from applying the modifiers to the molecular graph of the data structure.

Such an approach corresponds to enumerating the structural variants covered by the data structure and checking the structural variants one after the other.

It is also possible to take a step-by-step approach: In a first step, exact matching is carried out on the basis of the molecular graph of the data structure. In this step, those vertices (atoms) that may be affected by a modifier are not taken into account (so only a sub-graph or several sub-graphs are checked).

In other words, it is checked whether any sub-graph of the data structure that is not affected by any modifier occurs in the molecular graph of the query compound. This corresponds to a substructure search. Substructure searches can be performed using a subgraph isomorphism algorithm, for example. One example for subgraph isomorphism is Ullmann's algorithm (J.R. Ullmann: An Algorithm for Subgraph Isomorphism, Journal of the ACM (JACM), Volume 23, Issue 1, Pages 31 - 42).

Another subject of the present disclosure is thus a computer-implemented method of searching a query compound. The method comprises:
- providing a molecular graph of the query compound,
- providing a data structure according to the present disclosure, wherein the data structure comprises a molecular graph of a chemical compound,
- identifying those subgraphs of the molecular graph of the chemical compound that do not contain an atom that is affected by a modifier,
- checking whether the molecular graph of the query compound includes the identified subgraphs.

If the molecular graph of the query compound does not include the identified subgraphs, the structure represented by the molecular graph of the query compound cannot match any structural variant of the data structure. The search can be terminated. No further structural variants need to be checked.

If the molecular graph of the query compound includes the identified subgraph, structural variants of the data structure need to be checked.

The data structure of the present disclosure thus acts as a filter that accelerates a search by sorting out irrelevant data structures. This speeds up structure searches.

In the event that the data structure includes a substitution modifier, it is possible to treat those vertices (atoms) that are affected by the substitution modifier as non-labelled vertices. Treating them as non-labelled vertices means that a matching algorithm only checks whether a vertex is present, but not what kind of vertex (i.e. which atom) it is. This means that a partially unlabelled graph can be generated in which the atoms that may be affected by a substitution modifier are not labelled.

If a search shows that the partially unlabelled molecular graph of the data structure or subgraphs thereof do not match the molecular graph of the query compound, the search can be terminated; none of the structural variants of the data structure can match the structure of the query compound.

Only if the partially unlabelled molecular graph or subgraphs thereof match the molecular graph of the query compound, further structural variants need to be examined.

The data structure of the present disclosure thus acts as a filter that accelerates a search by sorting out irrelevant data structures. This speeds up structure searches.

Another subject of the present disclosure is thus a computer-implemented method of searching a query compound. The method comprises:
- providing a molecular graph of the query compound,
- providing a data structure according to the present disclosure, wherein the data structure comprises a molecular graph of a chemical compound, and a substitution modifier,
- generating a partially unlabelled molecular graph or subgraphs thereof based on the molecular graph of the chemical compound and the substitution modifier, wherein atoms of the partially unlabelled molecular graph that are affected by the substitution modifier are unlabelled,
- checking whether the molecular graph of the query compound matches the partially unlabelled molecular graph or includes the subgraphs.

The data structure of the present disclosure relates to a partially elucidated structure of a chemical compound. The usual goal is to fully elucidate the structure of the chemical compound. The data structure of the present disclosure makes it clear where uncertainties exist in the structure. The data structure of the present disclosure makes it clear which area(s) of the structure require(s) clarification. The data structure of the present disclosure may provide clues as to which investigations are necessary to fully elucidate the structure.

If further investigations are carried out on a chemical compound and/or further information is obtained about the structure of the chemical compound, these may lead to an update and thus a change in the data structure.

The data structure of the present disclosure allows efficient access and/or modification of partially elucidated structures, which, for example, is crucial for performing computational tasks effectively. The data structure of the present disclosure enables the management of partially elucidated structures in a way that optimizes resources and processing time.

The data structure of the present disclosure may be of advantage in regulatory approval procedures of crop protection products. Regulatory agencies require a detailed chemical identification and characterization of the active ingredient in a crop protection product. This includes its chemical structure, isomers, impurities, and metabolites. Particularly in the case of impurities and metabolites, it can be difficult to determine the chemical structure with certainty.

Regulatory frameworks, such as those of the Environmental Protection Agency (EPA) in the United States or the European Food Safety Authority (EFSA) in the European Union, have specific data requirements for the registration of crop protection products. These requirements include detailed chemical and toxicological data. When full structural elucidation is not possible, the data submitted must convincingly demonstrate that the lack of complete structural information does not compromise the assessment of the product's safety and efficacy.

In cases where the full structure cannot be completely elucidated due to technical limitations and/or when dealing with complex natural products and/or complex samples, partially elucidated structures along with other available chemical data are submitted for review.

The data structure of the present disclosure is particularly well suited to represent partially elucidated structures. The data structure of the present disclosure represents the possible structural variants in a condensed and easy-to-process form. The data structure of the present disclosure is therefore an advantageous representation of active ingredients, impurities and/or metabolites when submitting documents for regulatory approval.

As described, the data structure of the present disclosure allows a quick and easy enumeration of chemical structures. Pre-rendered images (e.g., png, jpg, svg) and/or other representations can be generated from these chemical structures. These representations of partially elucidated structures can be made available for other software systems to use, e.g. to display the representation in a report, in a software GUI, on metabolite pathway websites, etc.

Another application relates to training of artificial intelligence models. Such a model may be configured to predict a metabolite structure. The predicted structure may be compared to a reference database containing partially elucidated structures in the form of the data structure of the present disclosure to evaluate if the prediction is correct. In case of partially elucidated structure, it is not a matter of an unambiguous right or wrong prediction, but rather of a "maybe", which can be handled appropriately in the scoring function.

The operations in accordance with the teachings herein may be performed by at least one computer system specially constructed for the desired purposes or general-purpose computer specially configured for the desired purpose by at least one computer program stored in a typically non-transitory computer readable storage medium.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g. smartphone); all these systems can be utilized for carrying out the computer-implemented method of the present disclosure.

The term "non-transitory" is used herein to exclude transitory, propagating signals or waves, but to otherwise include any volatile or non-volatile computer memory technology suitable to the application.

The term "computer system" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer system or processor. The term processing unit includes a single processor or a plurality of distributed or remote such units.

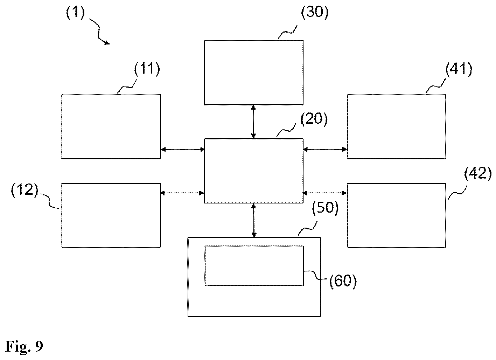

Fig. 9 illustrates a computer system (1) according to some example implementations of the present disclosure in more detail. The computer system may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs (60), which may be stored onboard the processing unit or otherwise stored in the memory (50) of the same or another computer.

The processing unit (20) may be a number of processors, a multi-core processor or some other type of processor, depending on the particular implementation. Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program (60) to perform one or more functions, the processing unit (20) of various examples may be capable of performing one or more functions without the aid of a computer program (60). In either instance, the processing unit (20) may be appropriately programmed to perform functions or operations according to example implementations of the present disclosure.

The memory (50) is generally any piece of computer hardware that is capable of storing information such as, for example, data, images, computer programs (e.g., computer-readable program code (60)), machine learning models and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s), camera(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display (30) may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers, cameras and the like.

As indicated above, a computer program (60) may be stored in memory (50), and executed by processing unit (20) that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

### Annex A

Example of an implementation of the data structure of the present disclosure (using the example of the chemical compound shown in Fig. 8(a))

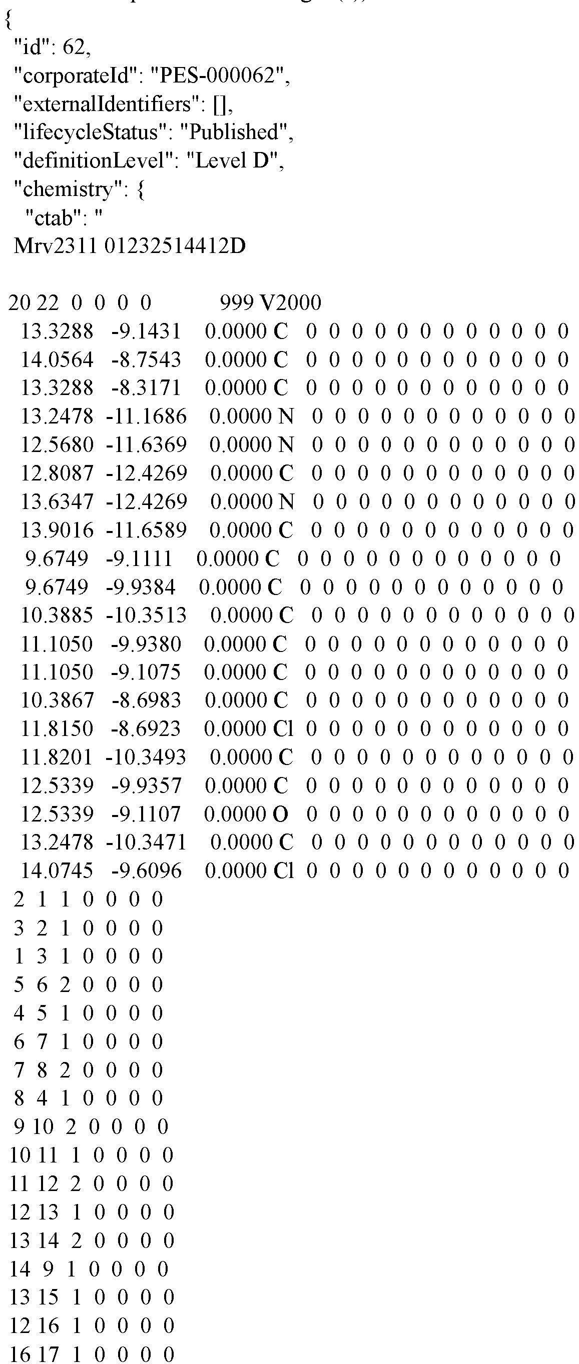

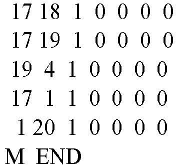

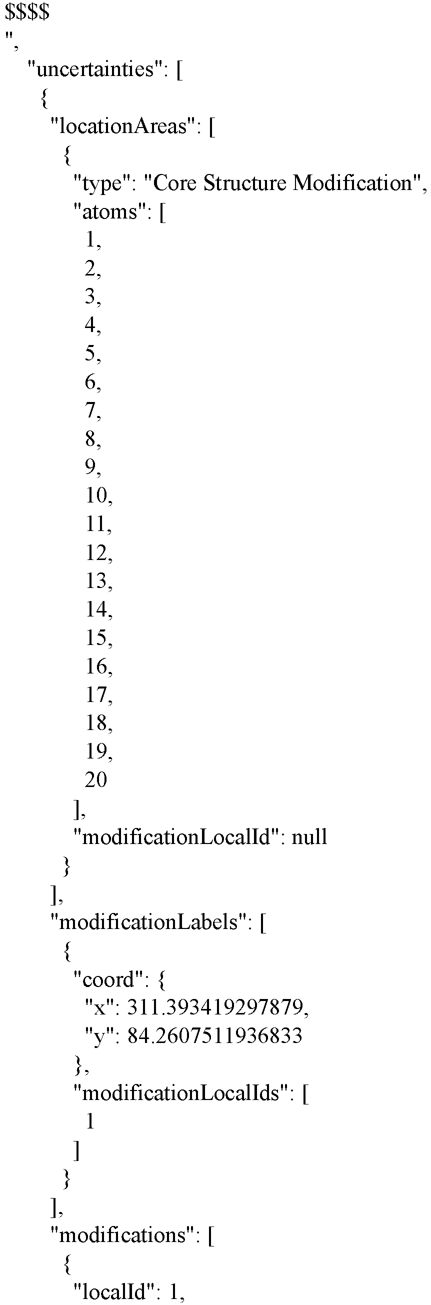

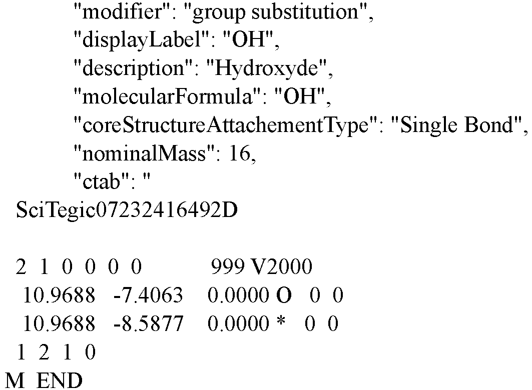

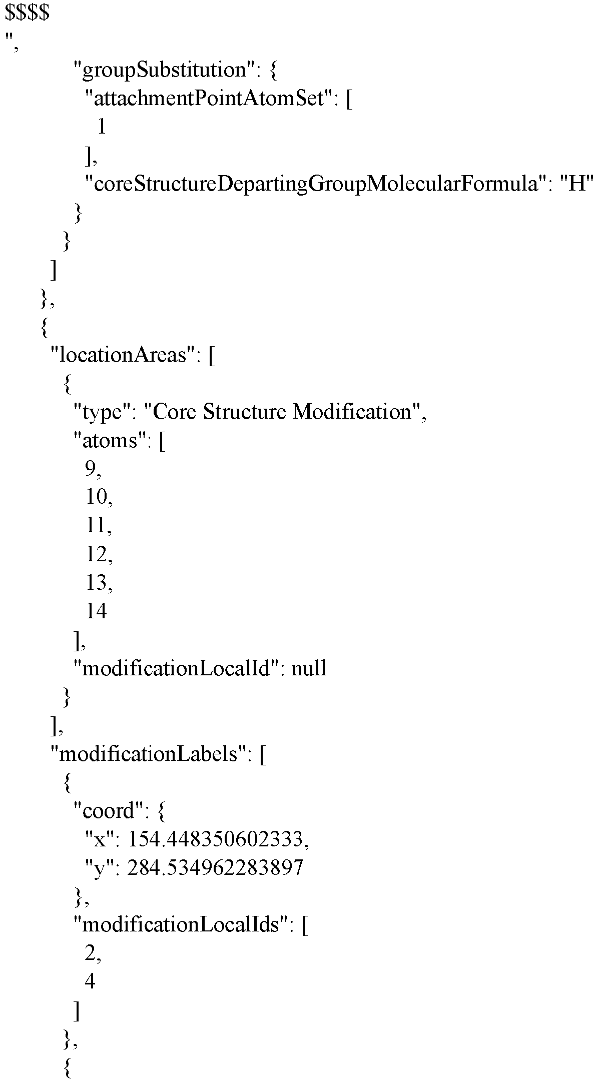

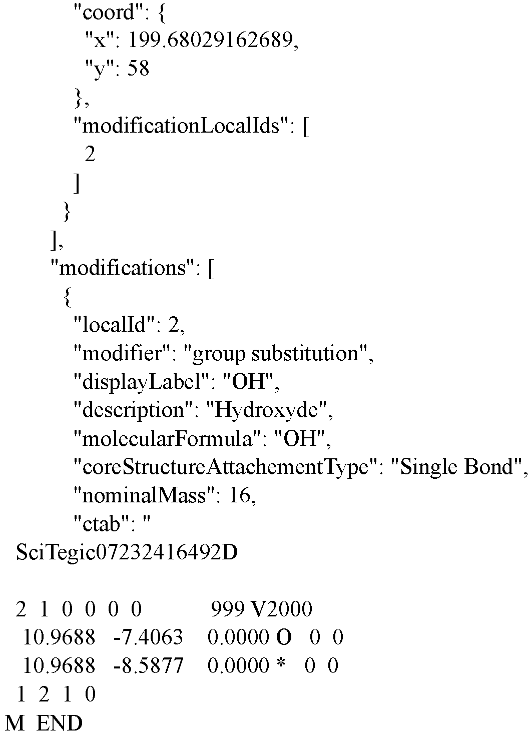

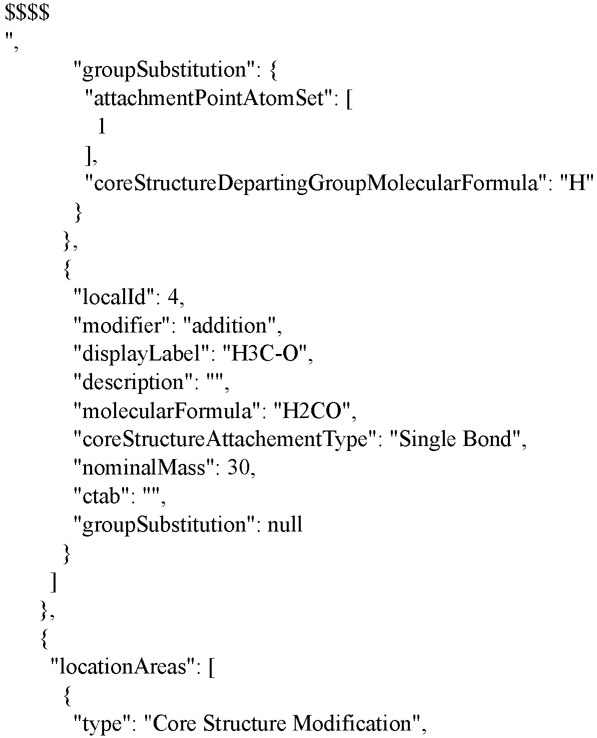

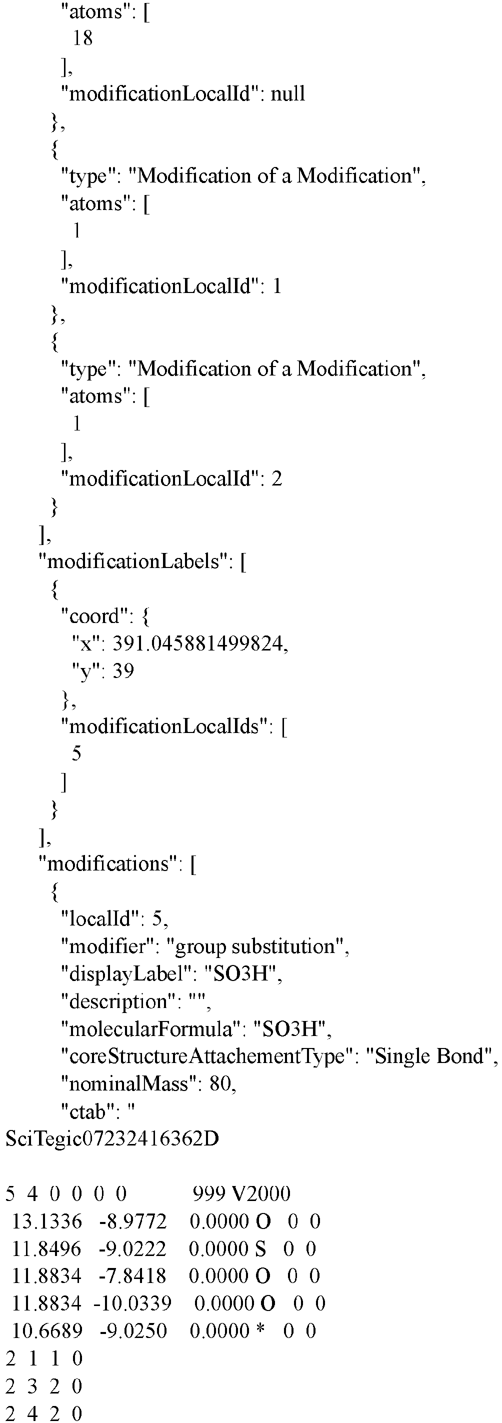

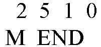

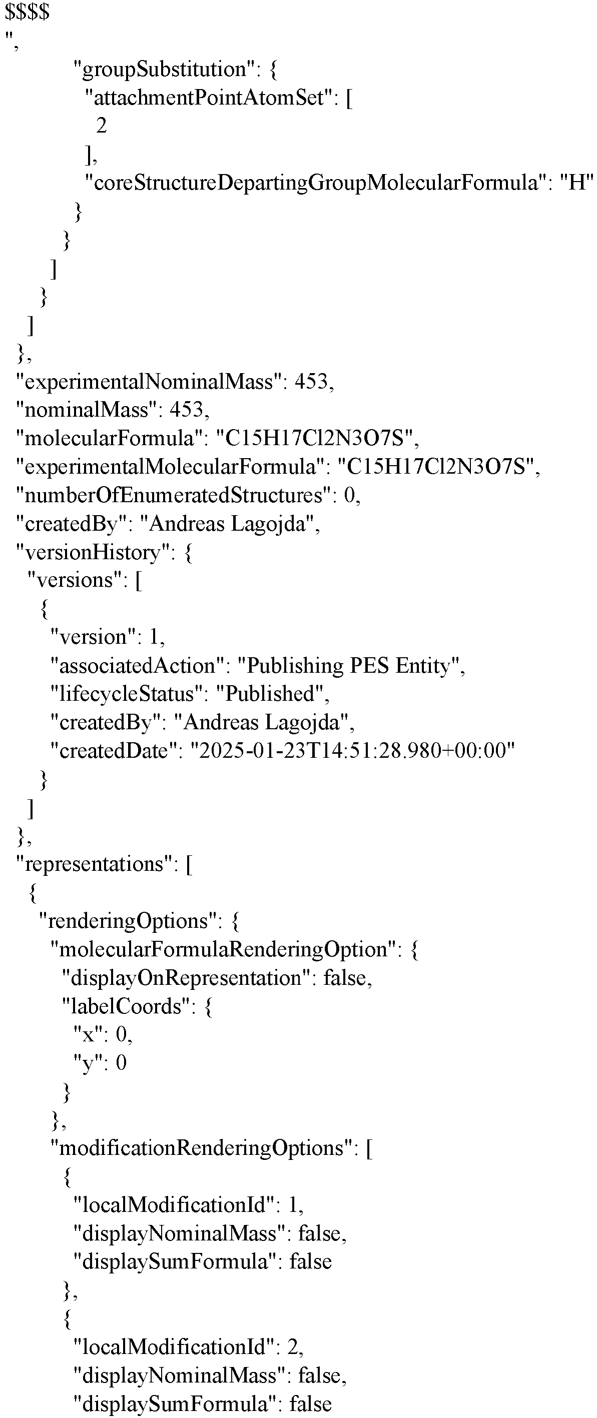

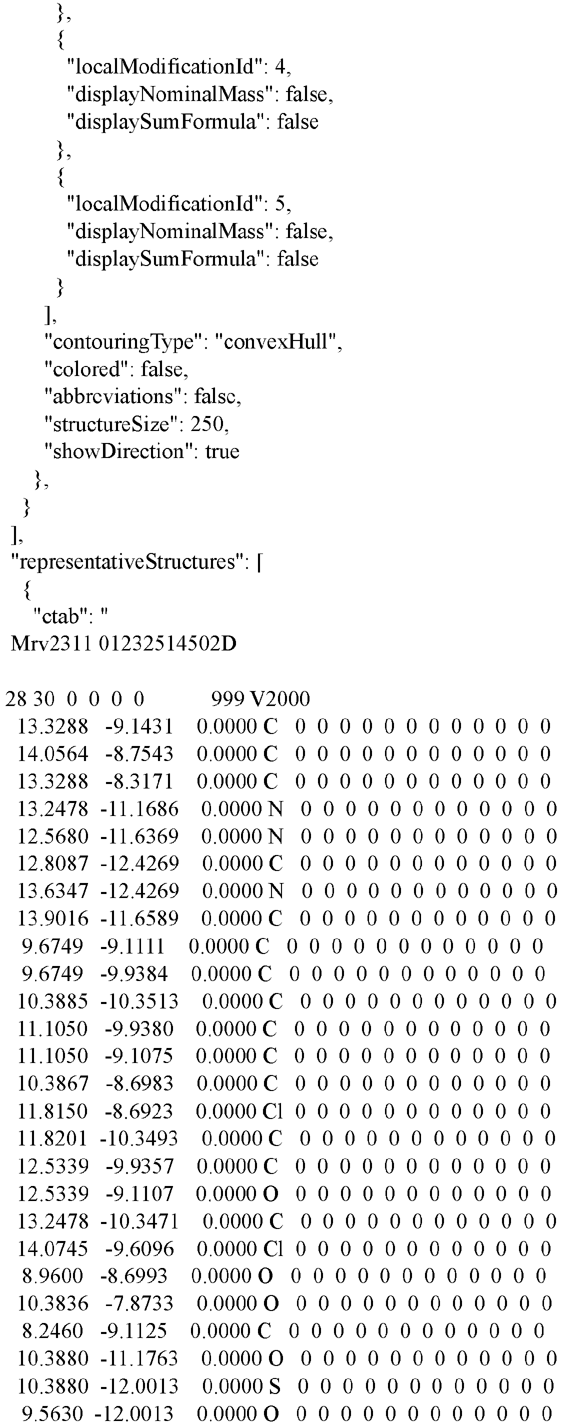

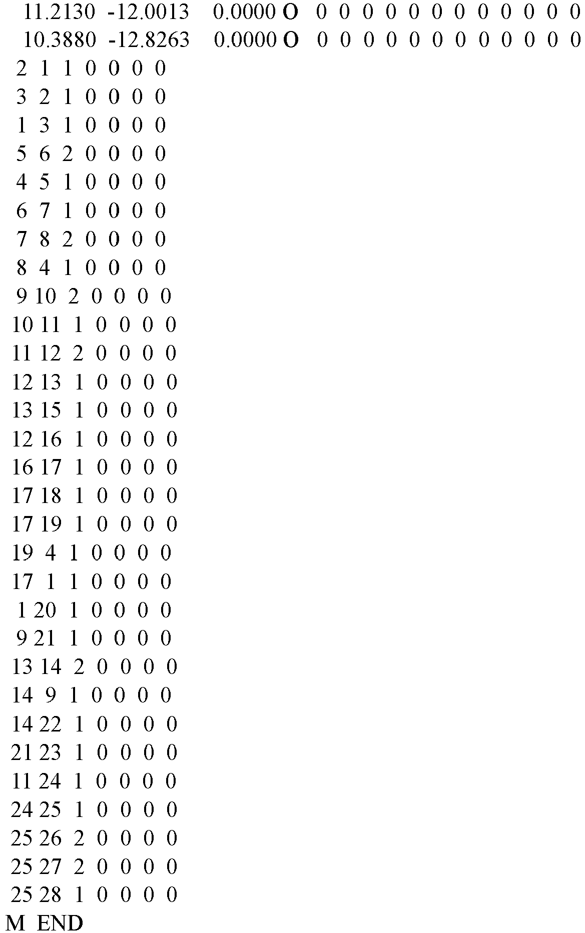

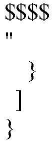

## Claims

1. A data structure for representing partially elucidated structures comprising:
- a graph defining atoms and bonds between the atoms of a chemical compound, and
- one or more modifiers selected from:
• substitution modifier
• addition modifier
• removal modifier
• rearrangement modifier
wherein each modifier defines the atoms and/or bonds of the graph affected by the modifier and how they are affected.

2. The data structure of claim 1,
wherein the substitution modifier indicates
- one or more first atoms or one or more first groups of atoms of the graph as well as one or more second atoms or one or more second groups of atoms by which the one or more first atoms or the one or more first groups atoms are replaced, or
- one or more first atoms of the graph, each of which is connected to a hydrogen atom, and one or more second atoms or one or more groups of atoms that replace the respective hydrogen atom(s), and/or
wherein the addition modifier indicates one or more first atoms and/or bonds of the graph as well as one or more second atoms or one or more groups of atoms to be added to the one or more first atoms and/or bonds, and/or
wherein the removal modifier indicates one or more first atoms and/or bonds of the graph to be removed from the graph, and/or
wherein the rearrangement modifier indicates at least two atoms and/or bonds of the graph that swap positions.

3. The data structure of any one of claims 1 to 2, further comprising a stereochemistry modifier, wherein the stereochemistry modifier indicates a graph's atom that represents a stereocenter and/or bonds attached to the atom, and one or more operations that convert the stereocenter to its opposite.

4. The data structure of any one of claims 1 to 3, further comprising an isotope modifier, wherein the isotope modifier indicates an atom of the graph and indicates which isotopes the atom can represent.

5. The data structure of any one of claims 1 to 4, wherein each modifier acting on the graph is a 1^{st}-order modifier, wherein the data structure comprises one or more *n*^{th}-order modifiers, whereby *n* is an integer greater than 1, whereby an *m*^{th}-order modifier acts on the graph resulting from the action of a (*m*-1)^{th}-order modifier, whereby *m* is an integer less than or equal to *n.*

6. The data structure of any one of claims 1 to 5, further comprising one or more of the following:
- a molecular formula
- a molecular mass
- an identification confidence level
- a level of definition
- review information
- confidentiality classification.

7. A computer-implemented method, the method comprising:
- receiving or providing a molecular graph;
- receiving an input from a user, the input specifying (i) atoms and/or bonds that are subject to one or more modifications, and (ii), information on the type of modification(s);
- generating a data structure based on the molecular graph and the user input;
- outputting the data structure and/or storing the data structure in a data storage and/or transmitting the data structure to a separate computer system.

8. The method of claim 7, further comprising:
- generating structural variants of the chemical compound represented by the data structure based on the data structure by combinatorially applying the modifiers to the molecular graph,
- outputting and/or saving the structural variants and/or transmitting the structural variants to a separate computer system.

9. The method of claims 7 or 8, further comprising:
- providing a data structure according to the present disclosure, wherein the data structure represents the chemical compound,
- generating structural variants of the chemical compound represented by the data structure based on the data structure by combinatorially applying the modifiers to the molecular graph,
- for each structural variant: computing chemical and/or physical properties based on the structural variant,
- comparing computed chemical and/or physical properties with measured chemical and/or physical properties of the chemical compound,
- selecting and/or discarding structural variants based on the result of the comparison.

10. The method of claim 9, further comprising:
- updating the data structure based on the selected and/or discarded structural variants,
- outputting and/or saving the updated data structure and/or transmitting the data structure to a separate computer system.

11. The method of any one of claims 7 to 10, further comprising:
- providing a data structure according to the present disclosure, wherein the data structure represents the chemical compound,
- providing one or more measured chemical and/or physical properties of the chemical compound represented by the data structure,
- computing one or more chemical and/or physical properties of one or more structural variants of the chemical compound based on the data structure,
- outputting
∘ the one or more computed chemical and/or physical properties and the one or more measured chemical and/or physical properties, and/or
∘ one or more deviations between the one or more computed chemical and/or physical properties and the one or more measured chemical and/or physical properties,
- receiving a selection and/or an exclusion by a user,
- updating the data structure based on the user's selection/exclusion,
- outputting and/or saving the updated data structure and/or transmitting the data structure to a separate computer system.

12. The method of any one of claims 7 to 11, further comprising:
- providing a molecular graph of a query compound,
- identifying those subgraphs of the molecular graph of the data structure that do not contain an atom that is affected by a modifier,
- checking whether the molecular graph of the query compound includes the identified subgraphs,
- outputting structural variants of the data structure of which the identified subgraphs are included in the molecular graph of the query compound.

13. The method of any one of claims 7 to 12, wherein the data structure comprises a substitution modifier, wherein the method further comprises:
- providing a molecular graph of a query compound,
- generating a partially unlabelled molecular graph or subgraphs thereof based on the molecular graph of the data structure and the substitution modifier, wherein atoms of the partially unlabelled molecular graph that are affected by the substitution modifier are unlabelled,
- checking whether the molecular graph of the query compound matches the partially unlabelled molecular graph or includes the subgraphs.
- outputting structural variants of the data structure
∘ of which the partially unlabelled molecular graph matches the molecular graph of the query compound, or
∘ of which the subgraphs are includes in the molecular graph of the query compound.

14. A computer system comprising:
a processing unit; and
a memory storing a computer program configured, when executed by the processing unit, to cause the computer system to perform the method of any one of claims 7 to 13.

15. A non-transitory computer readable storage medium having stored thereon a computer program that, when executed by a processing unit of a computer system, cause the computer system to perform the method of any one of claims 7 to 13.

16. Use of a data structure of any one of the claims 1 to 6 for representing and/or storing and/or processing partially elucidated structures and/or doing searches based on partially elucidated structures and/or calculations based on partially elucidated structures.

## Amended claims

### Amended claims in accordance with Rule 137(2) EPC.

1. A machine-readable data structure for representing partially elucidated structures comprising:
- a molecular graph defining atoms and bonds between the atoms of a chemical compound, and
- one or more modifiers selected from:
• substitution modifier
• addition modifier
• removal modifier
• rearrangement modifier
wherein each modifier defines the atoms and/or bonds of the molecular graph affected by the modifier and how they are affected,
wherein the substitution modifier indicates
- one or more first atoms or one or more first groups of atoms of the molecular graph as well as one or more second atoms or one or more second groups of atoms by which the one or more first atoms or the one or more first groups atoms are replaced, or
- one or more first atoms of the molecular graph, each of which is connected to a hydrogen atom, and one or more second atoms or one or more groups of atoms that replace the respective hydrogen atom(s),
wherein the addition modifier indicates one or more first atoms and/or bonds of the molecular graph as well as one or more second atoms or one or more groups of atoms to be added to the one or more first atoms and/or bonds,
wherein the removal modifier indicates one or more first atoms and/or bonds of the molecular graph to be removed from the molecular graph,
wherein the rearrangement modifier indicates at least two atoms and/or bonds of the molecular graph that swap positions.

2. The data structure of claim 1, wherein combinational application of the one or more modifiers on the molecular graph generates a plurality of structural variants of the chemical compound.

3. The data structure of any one of claims 1 to 2, further comprising a stereochemistry modifier, wherein the stereochemistry modifier indicates a molecular graph's atom that represents a stereocenter and/or bonds attached to the atom, and one or more operations that convert the stereocenter to its opposite.

4. The data structure of any one of claims 1 to 3, further comprising an isotope modifier, wherein the isotope modifier indicates an atom of the molecular graph and indicates which isotopes the atom can represent.

5. The data structure of any one of claims 1 to 4, wherein each modifier acting on the molecular graph is a 1^{st}-order modifier, wherein the data structure comprises one or more *n*^{th}-order modifiers, whereby n is an integer greater than 1, whereby an *m*^{th}-order modifier acts on the molecular graph resulting from the action of a (*m*-1)^{th}-order modifier, whereby *m* is an integer less than or equal to *n.*

6. The data structure of any one of claims 1 to 5, further comprising one or more of the following:
- a molecular formula
- a molecular mass
- an identification confidence level
- a level of definition
- review information
- confidentiality classification.

7. A computer-implemented method, the method comprising:
- receiving or providing a molecular graph;
- receiving an input from a user, the input specifying (i) atoms and/or bonds that are subject to one or more modifications, and (ii), information on the type of modification(s);
- generating a data structure of any one of claims 1 to 6 based on the molecular graph and the user input;
- outputting the data structure and/or storing the data structure in a data storage and/or transmitting the data structure to a separate computer system.

8. The method of claim 7, further comprising:
- generating structural variants of the chemical compound represented by the data structure based on the data structure by combinatorially applying the modifiers to the molecular graph,
- outputting and/or saving the structural variants and/or transmitting the structural variants to a separate computer system.

9. The method of claims 7 or 8, further comprising:
- providing a data structure of any one of claims 1 to 6, wherein the data structure represents the chemical compound,
- generating structural variants of the chemical compound represented by the data structure based on the data structure by combinatorially applying the modifiers to the molecular graph,
- for each structural variant: computing chemical and/or physical properties based on the structural variant,
- comparing computed chemical and/or physical properties with measured chemical and/or physical properties of the chemical compound,
- selecting and/or discarding structural variants based on the result of the comparison.

10. The method of claim 9, further comprising:
- updating the data structure based on the selected and/or discarded structural variants,
- outputting and/or saving the updated data structure and/or transmitting the data structure to a separate computer system.

11. The method of any one of claims 7 to 10, further comprising:
- providing a data structure of any one of claims 1 to 6, wherein the data structure represents the chemical compound,
- providing one or more measured chemical and/or physical properties of the chemical compound represented by the data structure,
- computing one or more chemical and/or physical properties of one or more structural variants of the chemical compound based on the data structure,
- outputting
o the one or more computed chemical and/or physical properties and the one or more measured chemical and/or physical properties, and/or
∘ one or more deviations between the one or more computed chemical and/or physical properties and the one or more measured chemical and/or physical properties,
- receiving a selection and/or an exclusion by a user,
- updating the data structure based on the user's selection/exclusion,
- outputting and/or saving the updated data structure and/or transmitting the data structure to a separate computer system.

12. The method of any one of claims 7 to 11, further comprising:
- providing a molecular graph of a query compound,
- identifying those subgraphs of the molecular graph of the data structure that do not contain an atom that is affected by a modifier,
- checking whether the molecular graph of the query compound includes the identified subgraphs,
- outputting structural variants of the data structure of which the identified subgraphs are included in the molecular graph of the query compound.

13. The method of any one of claims 7 to 12, wherein the data structure comprises a substitution modifier, wherein the method further comprises:
- providing a molecular graph of a query compound,
- generating a partially unlabelled molecular graph or subgraphs thereof based on the molecular graph of the data structure and the substitution modifier, wherein atoms of the partially unlabelled molecular graph that are affected by the substitution modifier are unlabelled,
- checking whether the molecular graph of the query compound matches the partially unlabelled molecular graph or includes the subgraphs.
- outputting structural variants of the data structure
∘ of which the partially unlabelled molecular graph matches the molecular graph of the query compound, or
∘ of which the subgraphs are includes in the molecular graph of the query compound.

14. A computer system comprising:
a processing unit; and
a memory storing a computer program configured, when executed by the processing unit, to cause the computer system to perform the method of any one of claims 7 to 13.

15. A non-transitory computer readable storage medium having stored thereon a computer program that, when executed by a processing unit of a computer system, cause the computer system to perform the method of any one of claims 7 to 13.

16. Use of a data structure of any one of the claims 1 to 6 for representing and/or storing and/or processing partially elucidated structures and/or doing searches based on partially elucidated structures and/or calculations based on partially elucidated structures.
